# EUROPEAN PATENT APPLICATION

(11) **EP 4 703 116 A1**
(43) Date of publication of application: **04.03.2026**
(21) Application number: 24207418.5
(22) Date of filing: 18.10.2024
(51) Int. Cl.: B29C 41/14, A41D 19/00, A61B 42/10, B29C 41/22, B29L 31/48

(54) **METHOD FOR MANUFACTURING HYPOALLERGENIC BIOTIC GLOVES AND HYPOALLERGENIC BIOTIC GLOVE**

(30) Priority: 27.08.2024 TW 113132255
(71) Applicant: Precious Mountain Ent. Corp., 1110 Tortola (VG)
(72) Inventor: HSUN HU, LIN HUANG, 114066 Tapei (TW)
(74) Representative: Straus, Alexander

(57) **Abstract**

A method for manufacturing biotic gloves and a hypoallergenic biotic glove are provided. The method includes dipping the molds into a coagulant solution and performing a pre-vulcanization treatment using alkaline protease, ultraviolet irradiation, and polyols derived from cellulose materials. The latex-coated molds undergo drying, washing, vulcanization with specific curing agents and accelerators, chlorine washing, and a secondary dipping in polyurethane to form a hypoallergenic inner layer. The resulting gloves feature a multilayer structure, including a first layer, a second layer, and a third layer. The first layer includes nitrile butadiene rubber, chloroprene rubber, or isoprene rubber combined with polysaccharide biotic materials. The second layer includes a natural rubber layer or other synthetic rubber combined with polysaccharide biotic materials, and the third layer includes a polyurethane layer. The final gloves have a biotic content of 3-40%, a tensile strength of 14-40 MPa, and an elongation at break of 350-800%.

## Description

### FIELD OF THE DISCLOSURE

The present invention relates to the field of disposable gloves, and more specifically, to a method for manufacturing biotic gloves with reduced protein extract content to lower the risk of allergic reactions in users.

### BACKGROUND OF THE INVENTION

Disposable gloves are widely used in various industries, including healthcare, food processing, and chemical handling, to provide a protective barrier against contaminants and harmful substances. Early disposable gloves were primarily made from natural rubber, which has long been associated with Type I allergic reactions caused by water-soluble proteins present in the latex. Additionally, accelerators and other chemicals used during the vulcanization process of natural rubber may cause Type IV chemical allergies.

Compared to natural rubber latex gloves, nitrile butadiene rubber (NBR) gloves are preferred for their superior oil resistance, chemical resistance, and puncture resistance. However, traditional NBR gloves tend to have less elasticity than natural rubber gloves.

Accordingly, there is a need for a glove and its manufacturing method that can provide the elasticity of natural rubber while effectively reducing protein extract content and allergenicity.

### SUMMARY OF THE INVENTION

To address the aforementioned problems, the primary objective of the present invention is to provide a method for manufacturing biotic gloves that significantly reduces protein extract content and allergenicity, thereby lowering the risk of allergic reactions in users. This is achieved through the use of specific proteases, ultraviolet (UV) irradiation, and advanced polymer formulations during the pre-vulcanization process, resulting in biotic gloves with enhanced safety and performance characteristics.

Another objective of the present invention is to enhance the structural integrity and performance of the gloves by using a multiple dipping technique that combines various polymer materials such as nitrile butadiene rubber (NBR), chloroprene rubber, isoprene rubber, natural rubber, and polyurethane, along with polysaccharide biotic materials. This multilayer structure is designed to improve the chemical resistance, puncture resistance, and abrasion resistance of the gloves.

A further objective of the present invention is to optimize the vulcanization process by using specific curing agents and accelerators to ensure that the gloves achieve the required mechanical properties, such as tensile strength and elongation, while maintaining a low protein extract content.

Another objective of the present invention is to simplify the manufacturing process by integrating effective washing and neutralization steps to remove residual chlorine and other soluble contaminants, ensuring that the final product is safe and hypoallergenic.

Based on the above objectives and others, the present invention provides a method for manufacturing biotic gloves that significantly reduces protein extract content and minimizes the risk of allergic reactions while maintaining good elasticity. The method incorporates several innovative steps and materials to enhance the safety and performance of the gloves.

The manufacturing process begins with cleaning the molds in a cleaning tank, followed by heating in an oven to remove moisture. The molds are then dipped into a coagulant solution in a coagulant dipping tank and heated in a coagulant oven to fix the coagulant onto the molds. The coagulant ensures that the latex subsequently dipped adheres and coagulates properly.

Next, the molds are dipped into a latex solution that has undergone pre-vulcanization treatment. This process includes the addition of alkaline protease at a concentration of 0.1% to 2.0%, a pH value of 9.5 to 10.5, and stirring at room temperature for 12 to 48 hours. The latex solution is also subjected to UV irradiation using lamps with a wavelength of 240-270 nm, an intensity of 1.0-15.0 mW/cm², and an exposure time of 5 seconds to 120 minutes. Additionally, polyols are incorporated into the latex solution at a concentration of 10-30% of the total solids, reacting with acid groups in the latex to form a cross-linked structure.

After the molds are dipped into the latex solution, the latex-coated molds are pre-dried in a pre-drying oven to remove moisture, washed with water in a pre-leaching tank for 60 to 300 seconds, and then dried in a drying oven. Next, the latex-coated molds are vulcanized at a temperature of 100-120°C for 18 to 25 minutes. The latex mixture includes curing agents and accelerators, such as inorganic oxides, sulfur, zinc dibutyldithiocarbamate, and zinc diethyldithiocarbamate. The concentration of inorganic oxides is 0.6-2 phr, sulfur is 0.6-2 phr, zinc dibutyldithiocarbamate is 0.2-1 phr, and zinc diethyldithiocarbamate is 0.2-1 phr.

After vulcanization, the gloves are cleaned in a chlorine washing tank with a chlorine concentration of 100-1000 ppm for 60 to 300 seconds, followed by rinsing with water to neutralize any residual chlorine. The gloves are then washed again in a post-leaching tank for 60 to 300 seconds. The next step involves dipping the gloves into a secondary material dipping tank containing 1-10% polyurethane (PU) polymer material, forming an inner layer with a thickness of approximately 0.01 mm or more. Finally, the gloves are dried and demolded, completing the manufacturing process.

The present invention provides a scalable and cost-effective solution for producing high-quality, hypoallergenic nitrile rubber gloves suitable for a wide range of applications. It overcomes the limitations of existing methods and offers enhanced safety and performance features.

### BRIEF DESCRIPTION OF THE DRAWINGS

FIG. 1 is a flowchart illustrating the manufacturing process of the biotic glove according to the present invention.
FIG. 2 is a flowchart illustrating the mold cleaning process according to the present invention.
FIG. 3 is a flowchart illustrating the heating process according to the present invention.
FIG. 4 is a flowchart illustrating the coagulant dipping process according to the present invention.
FIG. 5 is a flowchart illustrating the heating process according to the present invention.
FIG. 6 is a flowchart illustrating the process of dipping the mold into the latex solution according to the present invention.
FIG. 7 is a flowchart illustrating the pre-vulcanization process according to the present invention.
FIG. 8 is a schematic diagram illustrating the structure of the biotic glove in one embodiment of the present invention.

### DESCRIPTION OF THE PREFERRED EMBODIMENTS

The present invention relates to a new method for manufacturing biotic gloves, aiming to significantly reduce protein extract content and minimize the risk of allergic reactions. Traditional nitrile butadiene rubber (NBR) gloves are widely used in various industries, including healthcare, food processing, and chemical handling, due to their superior oil resistance, chemical resistance, and puncture resistance. However, despite these advantages, traditional NBR gloves may still pose an allergy risk to some users due to residual proteins and chemicals from the manufacturing process. Additionally, traditional NBR gloves tend to have poorer elasticity.

In the context of the present invention, "protein extract content" refers to the amount of extractable proteins in the gloves. Extractable proteins are those that can leach out from the glove material when in contact with aqueous solutions or during typical use. These proteins are of particular concern because they may come into direct contact with the user's skin, potentially causing allergic reactions, especially for individuals sensitive to natural rubber latex proteins. The manufacturing process for the hypoallergenic NBR biotic gloves of the present invention is carefully designed to minimize protein extract content. This is achieved through a series of pre-vulcanization treatments (detailed below), including the use of alkaline protease and ultraviolet (UV) irradiation. These steps are specifically intended to break down and remove proteins that could be extracted during use, thereby reducing the allergenic potential of the gloves. The effectiveness of these treatments in reducing protein extract content has been rigorously tested and verified according to industry standards, such as ASTM D5712. The results demonstrate a significant reduction in the protein extract content of the gloves produced by this invention, confirming the low-allergenicity of the final product. Reducing protein extract content is a critical aspect of the invention, as it directly impacts the hypoallergenic properties of the gloves. By targeting extractable proteins, this process ensures that the gloves are safer for users, particularly in sensitive environments such as healthcare and food processing.

The manufacturing method described in the present invention improves the safety and performance of the gloves by combining specific steps and materials. Key innovations include the use of alkaline protease, UV irradiation, and polyols during the pre-vulcanization process. Additionally, a multilayer structure is utilized to combine the advantages of synthetic and natural rubber, thereby enhancing the mechanical performance and hypoallergenic characteristics of the gloves.

Furthermore, the method employs a secondary dipping process with polyurethane to form an inner layer that comes into contact with the user's skin, further reducing the risk of Type IV chemical allergies caused by accelerators and other chemicals used in the vulcanization process. This inner layer acts as a barrier, enhancing the comfort and safety of the gloves for users with sensitive skin.

In addition to reducing allergenic potential, the method of the present invention also aims to optimize the mechanical performance of the gloves. The reaction of polyols with acid groups in the latex forms a cross-linked structure, increasing the tensile strength and elongation of the final product. The multilayer dipping technique further reinforces the gloves, making them suitable for demanding applications.

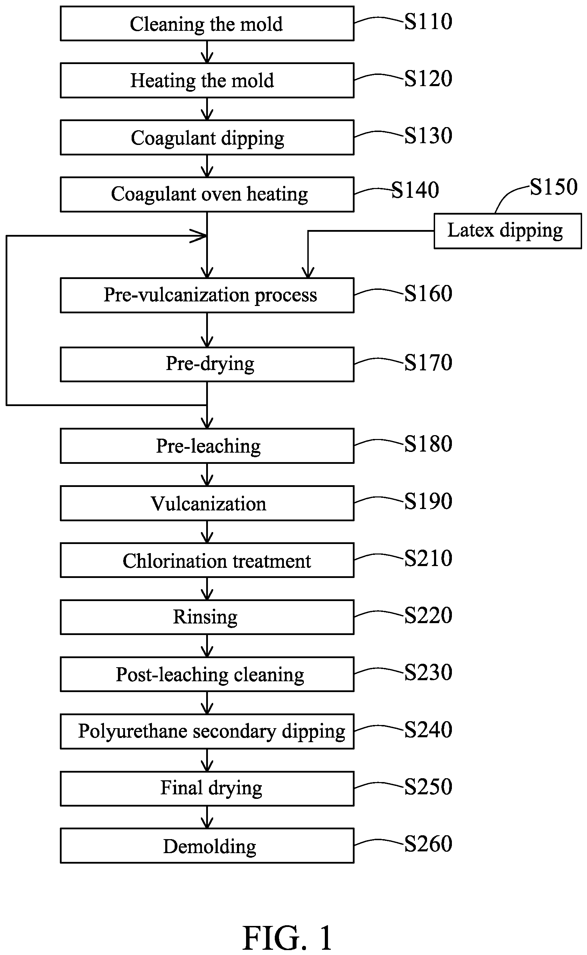

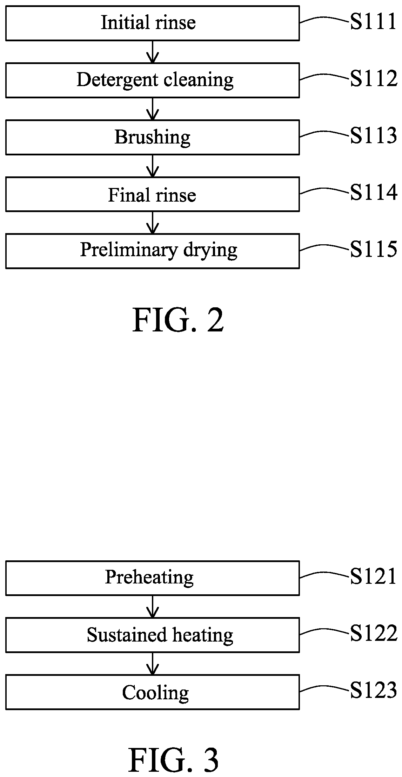

Referring to FIG. 1, which illustrates a flowchart of the manufacturing process for the biotic glove according to the present invention. This manufacturing process begins with the preparation of the mold and the pre-vulcanization of the latex. Proper mold preparation is essential to ensure that the surface of the biotic glove is smooth and free of defects and that the latex adheres uniformly to the mold during the manufacturing process. The mold preparation process involves two main steps: cleaning the mold (step S 110) and heating the mold (step S120) to remove moisture. Before the mold is dipped into the coagulant and latex solution, it must be thoroughly cleaned to remove any contaminants that could affect the quality of the gloves. As shown in FIG. 2, the mold cleaning process in this embodiment typically includes the following steps:
- S111 (Initial Rinse): First, the mold is rinsed with water to remove any loose particles and surface dust.
- S112 (Detergent Cleaning): The mold is then cleaned with a detergent solution to remove grease, oil, and other organic residues. This step ensures that the mold surface is free of contaminants that could interfere with latex adhesion.
- S113 (Brushing): The mold is brushed either manually or using mechanical brushes to ensure that all residues are thoroughly removed, especially in areas that are difficult to clean with rinsing alone.
- S114 (Final Rinse): After brushing, the mold is thoroughly rinsed with clean water to remove any remaining detergent and residues.
- S115 (Preliminary Drying): The cleaned mold needs to be preliminarily dried, which can be achieved by air drying naturally or using forced air drying.

After the mold is cleaned and dried, it must be heated to remove any remaining moisture (i.e., step S120). This step S120 is used to prevent defects in the latex coating and to ensure that the latex adheres properly to the mold. As shown in FIG. 3, the heating process generally includes the following steps:
- S121 (Preheating): The mold is placed in an oven and preheated to a specific temperature to ensure that all moisture is evaporated. The preheating temperature and time are adjusted on a case-by-case basis to achieve the desired results.
- S122 (Sustained Heating): The temperature is typically set between 50°C and 100°C, depending on the requirements of the manufacturing process. The mold is heated for a sufficient amount of time to ensure complete drying.
- S123 (Cooling): After heating, the mold may be allowed to cool slightly before proceeding to the next step in the manufacturing process. This cooling period helps stabilize the mold's temperature, ensuring uniformity in the subsequent latex coating.

Once the mold preparation is completed, the coagulant dipping process begins, which ensures that the latex film formed in subsequent steps adheres properly to the cleaned and heated mold. The coagulant dipping process involves two main stages: dipping the mold into the coagulant solution (step S130) and heating the mold in a coagulant oven (step S140).

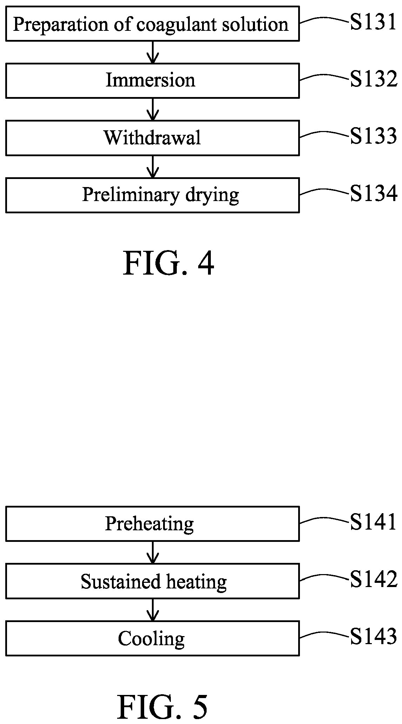

The purpose of step S130 is to promote the coagulation of the latex applied in subsequent steps, forming a uniform latex film that will become the glove. As shown in FIG. 4, the coagulant dipping process includes the following steps:
- S131 (Preparation of Coagulant Solution): The coagulant solution typically contains calcium nitrate dissolved in water or an alcohol solution. The concentration of calcium nitrate is carefully controlled to ensure effective coagulation and the formation of a uniform film. The coagulant solution may also include additives, such as surfactants or wetting agents, to improve the wettability of the mold and the uniformity of the coating.
- S132 (Dipping): The mold is dipped into the coagulant solution. Those skilled in the art can control the dipping time to ensure that the mold is evenly coated with the coagulant. Typically, the dipping time of the mold varies from a few seconds to a few minutes, depending on the desired thickness of the coagulant layer and specific process parameters.
- S133 (Withdrawal): The mold is slowly withdrawn from the coagulant solution at a controlled speed to ensure the formation of a uniform coating on the surface of the mold. The withdrawal speed can be adjusted to affect the thickness of the coagulant layer, with a slower withdrawal speed generally resulting in a thicker coating.
- S134 (Preliminary Drying): After dipping, the mold may undergo a brief preliminary drying period to stabilize the coagulant layer before proceeding to the next step. Preliminary drying can be accelerated by environmental air or forced air.

After completing step S130, the mold is heated in the coagulant oven to activate the coagulant and prepare the surface for latex adhesion in subsequent steps. As illustrated in FIG. 5, the heating process includes the following steps:
- S141 (Preheating): The mold is placed in the coagulant oven and preheated to a specific temperature. This preheating step ensures that the coagulant layer is properly dried and activated, preparing it for the subsequent latex dipping process.
- S142 (Sustained Heating): The temperature in the coagulant oven is maintained within a controlled range, typically between 50°C and 100°C, to ensure optimal drying and activation of the coagulant. The duration of heating is also controlled, typically lasting from several minutes to an hour, depending on the process requirements and the thickness of the coagulant layer. During the entire heating process, temperature and time are monitored and adjusted as necessary to ensure uniform activation of the coagulant. This ensures that the latex will adhere properly to the mold in the next step.
- S143 (Cooling): After heating for a specified duration, in some embodiments, the mold is allowed to cool slightly before proceeding to the latex dipping process. This cooling period helps stabilize the coagulant layer, ensuring uniform formation of the latex film in subsequent steps.

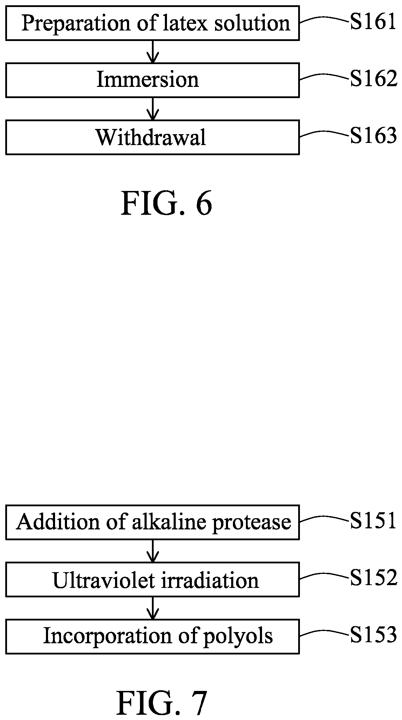

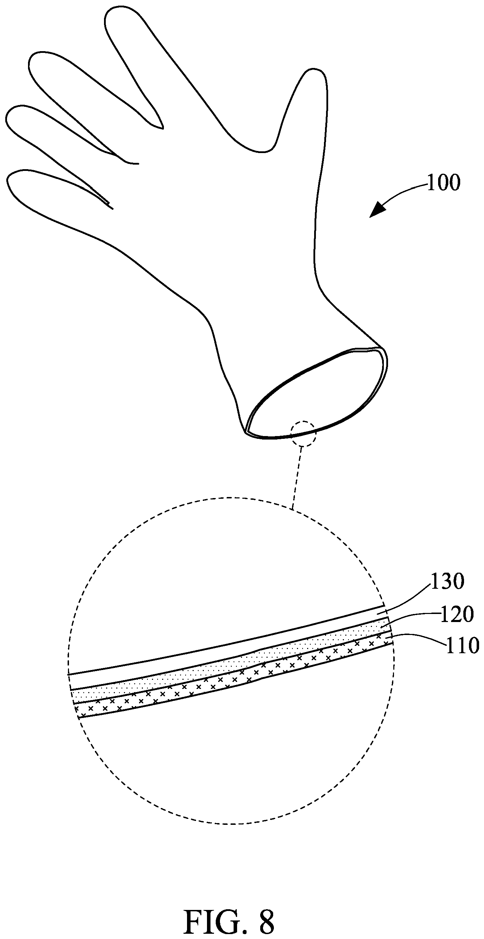

Next, the process involves latex dipping (step S160) and pre-drying (step S170). It is important to note that in this embodiment, steps S 160 and S 170 are repeated twice to form the first layer 110 and the second layer 120 of the biotic glove 100, as shown in FIG. 8. The purpose of step S 160 is to form a uniform latex layer on the mold and enhance the mechanical properties of the glove. Referring to FIG. 6, the process of dipping the mold into the latex solution includes the following steps:
- S161 (Preparation of Latex Solution): In the initial execution of step S160, the latex solution is prepared by mixing nitrile butadiene rubber latex with polysaccharide biotic materials and various additives to achieve the desired properties. In other embodiments, the latex solution may consist of other synthetic rubbers, such as chloroprene rubber and isoprene rubber, combined with polysaccharide biotic materials. In the second execution of step S160, the latex solution is prepared by mixing natural rubber latex with various additives to achieve the desired properties. These additives may include stabilizers, surfactants, and other compounds to improve the stability and uniformity of the latex. Additionally, the latex solution is thoroughly homogenized to ensure consistency in its composition.

- S162 (Dipping): After heating and coating with the coagulant, the mold is dipped into the latex solution. The dipping time is controlled to achieve the desired thickness of the latex layer. Typically, the dipping time for the mold varies from a few seconds to a few minutes.
- S163 (Withdrawal): The mold is withdrawn from the latex solution, and the withdrawal speed is controlled to affect the thickness and uniformity of the latex layer.

Subsequently, step S170 is performed. In one embodiment, step S170 involves heating the mold in a pre-drying oven to remove excess moisture from the latex-coated mold. In this step, the mold is placed in a pre-drying oven set at a temperature typically ranging from 50°C to 80°C, a temperature range sufficient to evaporate moisture without degrading the latex layer. The drying time typically lasts from 5 to 30 minutes, depending on the thickness of the latex layer and process requirements. After completing step S170, step S160 is executed a second time, with the main component of the latex solution being natural rubber latex in this second execution. It is important to note that whether step S160 is performed the first or second time, the latex solution used must undergo the pre-vulcanization process (step S150) to remove proteins, particularly since natural rubber contains a higher amount of proteins. Referring to FIG. 7, the pre-vulcanization process (step S150) includes the following steps:
- S151 (Addition of Alkaline Protease): This step can occur before the mold is dipped into the latex solution, where alkaline protease is added to the latex solution at a concentration of 0.1% to 2.0% by weight. Protease helps break down proteins in the latex, reducing the allergenicity of the final glove. Additionally, the pH of the latex solution is adjusted to a range of 9.5 to 10.5 to optimize the activity of the alkaline protease. This pH range ensures that the protease effectively breaks down proteins during the pre-vulcanization process. Furthermore, the latex solution with added protease is stirred at room temperature for 12 to 48 hours. This extended stirring period ensures that the protease has sufficient time to act on the proteins, further reducing their content in the final product.
- S152 (Ultraviolet Irradiation): The latex-coated mold is subjected to ultraviolet (UV) irradiation using UV lamps with a wavelength range of 240-270 nm. This specific wavelength range effectively breaks down proteins and other organic compounds in the latex. The intensity of UV irradiation is controlled between 1.0 and 15.0 mW/cm². The irradiation time can vary from 5 seconds to 120 minutes, depending on the desired level of protein reduction and other process parameters. It is also noteworthy that in other embodiments, the entire latex solution may be irradiated before the mold is dipped into it, depending on the requirements.
- S153 (Incorporation of Polyols): This step occurs before the mold is dipped into the latex solution, where polyols are incorporated into the latex solution at a concentration of 10-30% of the total solids in the latex solution. These polyols react with acid groups in the latex to form a cross-linked structure that enhances the mechanical properties of the glove. In one embodiment, the polyols chemically react with carboxylic acid groups in the latex to form ester bonds, cross-linking the latex molecules. This cross-linking forms a network structure within the latex, improving its tensile strength, elasticity, and resistance to water-soluble protein leaching. In this embodiment, the polyols are derived from cellulose materials.

Continuing to refer to FIG. 1, after completing the latex dipping, pre-vulcanization, and pre-drying processes, the pre-leaching step (step S180) is performed. In this step, the mold is cleaned in a pre-leaching tank to remove water-soluble proteins and other impurities. During step S 180, the mold is immersed in a cleaning solution in the pre-leaching tank, which is typically plain water. In some cases, additional reagents, such as surfactants or mild detergents, may be added to enhance the removal of impurities. The mold is cleaned in the pre-leaching tank for 60 to 300 seconds, with the specific duration depending on the level of impurities and the requirements of the manufacturing process. Additionally, the cleaning solution may be gently stirred or agitated to improve the efficiency of protein and impurity removal. This ensures that all surfaces of the latex-coated mold are thoroughly cleaned. In this step S180, the cleaning solution is typically maintained at a temperature between 20°C and 40°C, which helps increase the solubility of proteins and other contaminants, facilitating their removal.

After completing the pre-drying and pre-leaching steps, the vulcanization process (step S190) is initiated. The vulcanization process involves heating the latex-coated mold in the presence of curing agents and accelerators to form cross-links between the rubber molecules, thereby enhancing the elasticity, strength, and durability of the gloves. In this step S 190, the latex-coated mold is placed in a vulcanization oven, where the temperature is carefully controlled to ensure that the vulcanization process proceeds efficiently without causing degradation of the latex or other materials. In this embodiment, the temperature is typically maintained between 100°C and 120°C. The mold is heated for a duration ranging from 18 to 25 minutes in this embodiment. Of course, those skilled in the art may adjust the heating time based on the thickness of the latex layer, the composition of the latex mixture, and other process parameters.

During the vulcanization process, specific curing agents and accelerators are added to the latex to promote cross-linking of the rubber molecules. These substances typically include the following compounds:
Inorganic Oxides: In this embodiment, the concentration of inorganic oxides is 0.6-2 phr. Inorganic oxides enhance the stability and mechanical properties of the rubber by promoting effective cross-linking.

Sulfur: Sulfur is the primary curing agent, and in this embodiment, its concentration is 0.4-2 phr. It forms cross-links between rubber molecules, significantly improving the elasticity and durability of the gloves.

Zinc Dibutyldithiocarbamate (ZDBC): ZDBC acts as a secondary accelerator, and in this embodiment, its concentration is 0.2-1 phr. It accelerates the vulcanization process, reducing the time required for complete cross-linking and improving the efficiency of the process.

Zinc Diethyldithiocarbamate (ZDEC): ZDEC is another secondary accelerator, and in this embodiment, its concentration is 0.2-1 phr. Similar to ZDBC, it speeds up the vulcanization process, ensuring uniform cross-linking within the latex matrix.

The curing agents and accelerators are thoroughly mixed into the latex solution before the mold dipping process (step S160). This ensures that these compounds are evenly distributed within the latex matrix, promoting consistent vulcanization across all parts of the glove.

Next, continuing to refer to FIG. 1, the chlorination treatment (step S210) and washing (step S220) after vulcanization are performed to remove residual proteins, chemicals, and other contaminants that may remain after vulcanization, further reducing the risk of allergic reactions and ensuring the cleanliness and safety of the final product. In step S210, the purpose of the chlorination step is to remove residual proteins and other contaminants from the surface of the gloves. Chlorination is particularly effective in denaturing proteins, rendering them insoluble in latex and easier to wash away, which helps reduce the allergenic potential of the gloves. In this embodiment, the chlorine solution is prepared by dissolving chlorine in water, with a concentration typically ranging from 100 to 1000 ppm. This concentration is sufficient to denature the proteins without damaging the latex material.

In step S210, the vulcanized gloves, along with the molds, are immersed in the chlorine solution. The immersion time is carefully controlled to ensure thorough treatment. Typically, the gloves are immersed for 60 to 300 seconds. During the immersion process, the chlorine solution may be gently agitated to enhance the treatment effect and ensure that all glove surfaces are evenly exposed to the chlorine.

After the gloves have been immersed in the chlorine solution, step S220 is carried out, where the gloves undergo a rinsing process to neutralize the residual chlorine and remove any proteins and other contaminants dissolved in the chlorine solution. In this step, the gloves are rinsed with clean water to wash away the chlorine solution. Multiple rinse cycles may be performed to ensure the thorough removal of chlorine and other residues. In some cases, a neutralizing agent, such as sodium thiosulfate, may be added to the rinse water to ensure the complete neutralization of residual chlorine.

Subsequently, step S230 is performed, where the gloves undergo a final leaching process in a post-leaching tank to remove any remaining soluble contaminants, ensuring the final cleanliness of the gloves. This step further reduces the risk of allergic reactions by ensuring that the gloves are free of any residual chemicals and proteins. The post-leaching tank contains clean water, typically maintained at room temperature or slightly warm (20°C to 40°C), to enhance the solubility of the remaining contaminants. The cleaning process involves immersing the gloves in the cleaning solution for 60 to 300 seconds. The immersion time is controlled to ensure thorough cleaning. At the same time, the cleaning solution may be gently agitated or stirred to improve the efficiency of contaminant removal, ensuring that all surfaces of the gloves are thoroughly cleaned.

Next, step S240 is carried out, in which the gloves undergo a secondary dipping process to apply a layer of polyurethane to the inner surface of the biotic gloves, providing a smooth, non-irritating barrier that reduces the risk of Type IV chemical allergies and enhances overall user comfort. The polyurethane solution is prepared by dissolving or dispersing polyurethane polymer material in an appropriate solvent or aqueous medium. The concentration of the polyurethane solution is adjusted to achieve the desired characteristics of the inner layer coating. Typically, the polyurethane concentration in the solution ranges from 1% to 10% by weight, ensuring that the coating is uniform and effective, with good adhesion to the latex surface.

The secondary dipping process involves immersing the gloves, still on the molds, into the polyurethane solution. The immersion time is controlled to ensure that the inner surface of the gloves is uniformly coated with a thin layer of polyurethane. The immersion time typically ranges from a few seconds to a few minutes, depending on the desired thickness of the coating. The mold is slowly withdrawn from the polyurethane solution at a controlled speed, where the withdrawal speed affects the thickness and uniformity of the polyurethane layer. A slower withdrawal speed generally results in a thicker coating.

Additionally, in this embodiment, the formed polyurethane inner layer typically has a thickness of 0.01 to 0.04 millimeters. This thickness provides an effective barrier against allergens and irritants without significantly affecting the flexibility and tactile sensitivity of the gloves.

Following the previous steps, the drying step (step S250) and the demolding step (step S260) are performed to ensure that the gloves are fully dried and safely removed from the molds without compromising their integrity or quality. Step S250 ensures that any residual solvents or moisture from the secondary dipping process are removed. In this step, the gloves are placed in a drying oven or subjected to forced air drying, with the temperature controlled between 50°C and 70°C. This temperature range is sufficient to evaporate any remaining moisture or solvent without damaging the latex or polyurethane layers. The drying time is carefully controlled, typically lasting 20 to 60 minutes, depending on the glove's characteristics and the thickness of the layers. This duration ensures that the gloves are thoroughly dried and fully cured.

Proper air circulation is maintained within the drying oven to ensure that all gloves are dried evenly. This prevents localized overheating or under-drying, which could affect the quality and performance of the gloves. The drying process is monitored to ensure that the temperature and duration remain within the specified range. Any deviations are immediately corrected to ensure consistent quality.

Step S260 involves carefully removing the fully dried gloves from the molds. In manual processes, skilled workers carefully peel the gloves from the molds. This method requires precise handling to avoid tearing or stretching the gloves. In automated processes, mechanical equipment gently removes the gloves from the molds. Automation can improve efficiency and consistency while reducing the risk of damage.

In some cases, a small amount of release agent or lubricant may be used to facilitate the demolding process. This helps prevent the gloves from sticking to the molds and reduces the risk of tearing. Mechanical devices such as air jets or mechanical fingers can be used to assist in removing the gloves from the molds. These devices gently lift the edges of the gloves, making them easier to peel off. After demolding, the gloves undergo a thorough inspection to check for defects or damage. This includes inspecting for uniform thickness, tears, holes, and overall quality. Defective gloves are identified and removed from the production line. The remaining gloves are sorted and prepared for packaging.

Samples from each batch of gloves are tested to ensure they meet the specified standards for tensile strength, elongation, and hypoallergenic characteristics. This ensures that the gloves will perform as expected in their intended applications. The gloves are packaged according to industry standards and prepared for distribution. Proper packaging helps maintain the quality and hygiene of the gloves until they reach the end user.

Referring to FIG. 8, which illustrates a schematic diagram of one embodiment of the biotic glove 100 according to the present invention. As shown in FIG. 8, the biotic glove 100 has a multilayer structure, with the primary objectives of enhancing its mechanical performance, chemical resistance, and hypoallergenic properties. The composition, function, and thickness of each layer of the biotic glove 100 will be described in detail below.

In this embodiment, the multilayer structure of the biotic glove 100 includes a first layer 110, a second layer 120, and a third layer 130. The first layer 110 is primarily composed of nitrile butadiene rubber (NBR) combined with polysaccharide biotic materials. NBR is known for its excellent oil resistance, chemical resistance, and puncture resistance. Additionally, the first layer 110 may incorporate or blend other synthetic rubbers, such as chloroprene rubber and isoprene rubber, combined with polysaccharide biotic materials to achieve specific performance characteristics. The first layer 110 serves as the main structural component of the biotic glove 100, providing the necessary strength, durability, and chemical resistance. The use of NBR or other synthetic rubbers ensures that the biotic glove 100 can withstand harsh environments and chemical exposure, making it suitable for various industrial and medical applications.

The second layer 120 is primarily composed of natural rubber, which is selected for its superior elasticity, tensile strength, and comfort. It enhances the overall flexibility and fit of the biotic glove 100. The second layer 120 provides additional strength and elasticity to the glove, improving the comfort and fit for the wearer. The natural rubber layer also enhances tactile sensitivity, making the glove suitable for fine operations.

In other embodiments, the second layer 120 may also be composed of other synthetic rubbers combined with polysaccharide biotic materials. Synthetic rubbers, such as nitrile butadiene rubber (NBR) or chloroprene rubber, provide superior oil resistance, chemical resistance, and puncture resistance, making the gloves suitable for use in harsh industrial environments and chemical handling applications. The inclusion of polysaccharide biotic materials (e.g., cellulose or starch) enhances the environmental friendliness of the gloves, making them a more sustainable option. These biotic materials are biodegradable and help reduce the carbon footprint of the product. The combination of synthetic rubber with polysaccharide biotic materials also provides a balanced approach to maintaining the mechanical performance of the gloves. Polysaccharides can act as fillers or reinforcing agents, improving the strength and durability of the synthetic rubber layer. Such gloves are not only robust and durable but also lightweight and comfortable, suitable for prolonged wear. Moreover, the combination of synthetic rubber and polysaccharide biotic materials ensures low protein extract content, thereby reducing the risk of allergic reactions. This makes the gloves suitable for users who are sensitive to natural rubber proteins, expanding their application across various fields such as medical, food processing, and laboratory environments.

In summary, whether the second layer 120 is composed of natural rubber or a combination of synthetic rubber and polysaccharide biotic materials, it plays a crucial role in enhancing the overall performance of the gloves. It provides the necessary strength, elasticity, and comfort while meeting specific requirements for chemical resistance, sustainability, and hypoallergenic properties. This versatility ensures that the biotic glove 100 is suitable for a wide range of applications, offering high performance and user satisfaction.

In this embodiment, the third layer 130 is primarily composed of polyurethane, which is characterized by its hypoallergenic properties and its ability to form a smooth, non-irritating inner surface. The primary function of the third layer is to provide a hypoallergenic barrier, reducing the risk of Type IV chemical allergies. The third layer 130 enhances comfort for users with sensitive skin, providing a smooth inner surface that makes the biotic glove 100 easy to don and remove. The third layer 130 is formed during the secondary dipping process (step S240).

In this embodiment, the thickness of the first layer 110 typically ranges from 0.01 mm to 0.2 mm. This range ensures that the biotic glove 100 has sufficient strength and chemical resistance while maintaining flexibility. The thickness of the second layer 120 also ranges from 0.01 mm to 0.2 mm. The second layer 120 adds to the overall thickness of the glove, enhancing its elasticity and comfort without making it overly bulky. The thickness of the third layer 130 ranges from 0.01 mm to 0.04 mm. This layer is designed to be thin enough to maintain flexibility and comfort while providing an effective hypoallergenic barrier. The total thickness of the glove, combining all three layers, typically ranges from 0.03 mm to 0.44 mm. The multilayer structure of the biotic glove 100 in this embodiment balances the needs for strength, flexibility, and hypoallergenic properties, resulting in a high-performance glove suitable for a variety of applications.

Finally, the biotic gloves produced through this innovative manufacturing process exhibit a range of excellent properties, making them highly suitable for various demanding applications. The biotic content of the gloves ranges from 3% to 40%, reflecting the inclusion of renewable and biodegradable materials, such as polysaccharide biotic materials. This significant biotic content highlights the environmental sustainability of the gloves, meeting the growing market demand for eco-friendly products.

The tensile strength of the gloves ranges from 14 to 40 MPa, ensuring that the gloves possess the necessary mechanical strength to withstand rigorous use. This high tensile strength is crucial for applications that require gloves to endure high stress and stretching, such as in industrial environments, medical procedures, and laboratory work. The enhanced tensile strength also contributes to the durability of the gloves, reducing the frequency of glove replacements, thereby offering cost-effectiveness.

The elongation at break of the biotic gloves ranges from 350% to 800%, indicating excellent flexibility and elasticity. This characteristic is particularly important to ensure that the biotic gloves can stretch and conform to the user's hand without tearing, providing a comfortable and secure fit. A high elongation at break is vital for maintaining flexibility and tactile sensitivity, allowing users to perform delicate and precise tasks with ease.

A key feature of the biotic gloves is their low protein extract concentration, which is less than 50 ppm. This low concentration is achieved through a pre-vulcanization process, including the use of alkaline protease and ultraviolet (UV) irradiation, which effectively breaks down and eliminates extractable proteins. By reducing the protein extract content, the gloves significantly lower the risk of allergic reactions, making them suitable for individuals who are sensitive or allergic to latex. This hypoallergenic characteristic enhances the gloves' applicability in medical, food processing, and other sensitive environments.

The palm thickness of the gloves is greater than 0.03 mm, providing a balance between protection and tactile sensitivity. This thickness ensures that the gloves offer adequate barrier protection against contaminants and hazardous substances while maintaining the flexibility needed for performing fine motor tasks. The optimal palm thickness also contributes to the overall durability of the gloves, enabling them to withstand wear and tear in various applications.

In summary, the biotic gloves produced by this method exhibit a range of advanced performance characteristics:
Biotic Content: 3% to 40%, highlighting the use of renewable materials.
Tensile Strength: 14 to 40 MPa, ensuring mechanical strength.
Elongation at Break: 350% to 800%, providing excellent flexibility and elasticity.
Protein Extract Concentration: Less than 50 ppm, reducing the risk of allergic reactions.
Palm Thickness: Greater than 0.03 mm, balancing protection and tactile sensitivity.

These performance characteristics make the biotic gloves an ideal choice for various applications, offering high performance, safety, and sustainability. The innovative combination of materials and advanced manufacturing processes ensures that the gloves meet the stringent requirements of different industries, providing user comfort and environmental benefits.

To verify the hypoallergenic nature and environmental sustainability of the biotic gloves, the applicant commissioned two comprehensive test reports from third-party organizations. These reports provide valuable insights into the protein extract content and biobased carbon content of the gloves, confirming their suitability for sensitive users and eco-friendly applications.

The first test report was conducted by SGS Taiwan Ltd. (Report No.: HQ40011/2023), focusing on the water-soluble protein extract content in the biotic gloves of the present invention. Using the ASTM D5712-15 method, specifically the modified Lowry method, the analysis revealed that the protein extract content in the tested samples was 27.5 ppm. This low level of water-soluble extractable proteins indicates a reduced risk of allergic reactions from the biotic gloves. The test was conducted under controlled environmental conditions, with an ambient temperature of 25 ± 3°C and a relative humidity of 65 ± 10%, using a Shimadzu UV-1700 UV-VISIBLE spectrophotometer. The results confirm that the protein extract content of the biotic gloves in the present invention falls within an acceptable low range, making them suitable for individuals sensitive to latex.

The second test report was provided by Beta Analytic (Report No.: Beta-660692), evaluating the biobased carbon content of the biotic gloves. Using the ASTM D6866-22 Method B (AMS) TOC, the report indicated that the gloves contained 37% biobased carbon, meaning that 37% of the carbon content in the gloves originates from renewable biomass sources, such as plant or animal by-products, while the remaining 63% comes from fossil sources like petroleum. The measured percentage modern carbon (pMC) was 36.61 ± 0.12 pMC, and the test was adjusted using an atmospheric adjustment factor to reflect contemporary carbon dioxide levels. This significant proportion of biobased carbon emphasizes the environmental sustainability of the gloves and aligns with the growing demand for eco-friendly products. The analysis was conducted according to stringent standards, with the results certified by ISO/IEC 17025:2017 testing accreditation PJLA #59423, ensuring high precision and reliability.

These test reports collectively validate the dual advantages of the biotic gloves of the present invention: hypoallergenic properties and a considerable use of renewable materials. The SGS report confirms the extremely low protein extract content of the gloves, reducing the likelihood of allergic reactions, while the Beta Analytic report underscores the gloves' commitment to sustainability, demonstrating that a significant portion of their composition comes from natural sources. These findings make the biotic gloves of the present invention a premium choice for health-conscious and environmentally aware consumers, ensuring safety, comfort, and environmental responsibility across various applications.

## Claims

1. A method for manufacturing a biotic glove, comprising:
(a) providing a mold suitable for manufacturing gloves, wherein a coagulant is fixed to the surface of the mold;
(b) dipping the mold into a latex solution, followed by pre-drying the latex-coated mold to form a latex-coated mold;
(c) washing the latex-coated mold with water;
(d) drying the latex-coated mold;
(e) vulcanizing the latex-coated mold to cure the latex and form a glove;
(f) chlorinating the vulcanized glove to remove residual proteins and contaminants;
(g) rinsing the chlorinated glove to remove residual chlorine;
(h) dipping the glove into a secondary material dipping tank containing a polyurethane polymer material;
(i) drying the glove; and
(j) demolding the glove;
wherein, prior to performing step (e), steps (b) through (d) are performed at least twice; wherein, during the first performance of steps (b) through (d), the latex solution comprises synthetic rubber combined with a biotic polysaccharide, and during the second performance of steps (b) through (d), the latex solution comprises natural rubber, and wherein prior to performing steps (b) through (d), the latex solution undergoes a pre-vulcanization treatment that includes the addition of an alkaline protease.

2. The method for manufacturing a biotic glove of claim 1, wherein the alkaline protease is added in a concentration of 0.1% to 2.0% by weight, with a pH value of 9.5 to 10.5, and the latex solution is stirred at room temperature for 12 to 48 hours.

3. The method for manufacturing a biotic glove of claim 2, wherein the pre-vulcanization treatment includes exposing the latex solution to ultraviolet radiation.

4. The method for manufacturing a biotic glove of claim 1, wherein the ultraviolet radiation is performed using ultraviolet lamps with a wavelength of 240-270 nm, an intensity of 1.0-15.0 mW/cm², and an exposure time of 5 seconds to 120 minutes.

5. The method for manufacturing a biotic glove of claim 1, wherein the pre-vulcanization treatment includes incorporating polyols into the latex solution.

6. The method for manufacturing a biotic glove of claim 5, wherein the polyols are added at a concentration of 10-30% by weight of the total solids in the latex solution and react with acid groups in the latex to form a cross-linked structure.

7. The method for manufacturing a biotic glove of claim 6, wherein step (e) comprises vulcanizing the latex-coated mold at a temperature of 100-120°C for 18-25 minutes, and the latex includes curing agents and accelerators.

8. The method for manufacturing a biotic glove of claim 7, wherein the curing agents and accelerators include inorganic oxides, sulfur, zinc dibutyldithiocarbamate, and zinc diethyldithiocarbamate; wherein the concentration of inorganic oxides is 0.6-2 phr, sulfur is 0.6-2 phr, zinc dibutyldithiocarbamate is 0.2-1 phr, and zinc diethyldithiocarbamate is 0.2-1 phr.

9. The method for manufacturing a biotic glove of claim 1, wherein step (f) comprises soaking the glove in a chlorine solution with a concentration of 100-1000 ppm for 60-300 seconds.

10. The method for manufacturing a biotic glove of claim 1, wherein the secondary material dipping tank contains a polyurethane polymer material at a concentration of 1-10%, and step (h) forms an inner layer with a thickness of about 0.01-0.04 mm.

11. A hypoallergenic biotic glove, comprising:
a multilayer structure, the multilayer structure comprising a first layer, a second layer, and a third layer;
wherein the first layer comprises a combination of nitrile butadiene rubber,
chloroprene rubber, or isoprene rubber with a polysaccharide biotic material;
wherein the second layer comprises natural rubber or synthetic rubber combined with a polysaccharide biotic material;
wherein the third layer comprises a polyurethane inner layer; wherein the glove has a biotic content ranging from 3% to 40%, a tensile strength ranging from 14 to 40 MPa, an elongation at break ranging from 350% to 800%, a protein extract concentration of less than 50 ppm, and a palm thickness greater than 0.03 mm.

12. The hypoallergenic biotic glove of claim 11, wherein the first layer has a thickness ranging from 0.01 mm to 0.2 mm.

13. The hypoallergenic biotic glove of claim 11, wherein the second layer has a thickness ranging from 0.01 mm to 0.2 mm.

14. The hypoallergenic biotic glove of claim 11, wherein the third layer has a thickness ranging from 0.01 mm to 0.04 mm.

15. The hypoallergenic biotic glove of claim 11, wherein the polysaccharide biotic material in the first and second layers is selected from the group consisting of cellulose and starch.
